(19) Europäisches Patentamt
European Patent Office
Office européen des brevets

(11) Publication number : **0 295 886 B1**

(12) # EUROPEAN PATENT SPECIFICATION

(45) Date of publication of patent specification : **02.01.92 Bulletin 92/01**

(51) Int. Cl.$^5$ : **A61K 7/02,** A61K 7/48

(21) Application number : **88305453.8**

(22) Date of filing : **15.06.88**

(54) **Facial cleansers.**

(30) Priority : **16.06.87 JP 149778/87**

(43) Date of publication of application : **21.12.88 Bulletin 88/51**

(45) Publication of the grant of the patent : **02.01.92 Bulletin 92/01**

(84) Designated Contracting States : **BE DE FR GB**

(56) References cited :
**GB-A- 2 008 943**
**GB-A- 2 158 839**
**US-A- 3 645 904**
**Chemical Abstracts, vol. 109, no. 20, 14 November 1988, page 364, column 1, abstract no.176091p, Colombus, Ohio, USA; T. Nanba et al.: "Silicone rubber for skin cosmetics" & JP-A-62 298518, 25.12.1987**

(73) Proprietor : **TORAY SILICONE COMPANY, LIMITED**
**2-8 Nihonbashi Muromachi Chuo-ku**
**Tokyo 103 (JP)**

(72) Inventor : **Harashima, Asao**
**6, 1-chome, Yushudai Nishi**
**Ichihara-shi, Chiba Prefecture (JP)**
Inventor : **Yoshida, Keiji**
**6, 1-chome, Yushudai Nishi**
**Ichihara-shi, Chiba Prefecture (JP)**

(74) Representative : **Lewin, John Harvey et al**
**Elkington and Fife Prospect House 8**
**Pembroke Road**
**Sevenoaks, Kent TN13 1XR (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a facial cleanser, and more specifically relates to a facial cleanser which contains powdered organopolysiloxane elastomer.

The following materials have been compounded in facial cleansers for the purpose of effectively removing foundation and make-up cosmetics : plant powders such as those obtained from hydrogenated jojoba oil and hydrogenated coconut oil, as well as the seeds and powders obtained from the apricot, almond, birch, walnut, peach, corn, sunflower, watermelon, etc. ; powders of materials of animal origin such as powdered crab shell, powdered eggshell, etc., as well as those obtained from hydrogenated beef tallow and hydrogenated lard ; organic powders such as those obtained from polyethylene, nylon, polypropylene, polyvinyl chloride, polystyrene, and cellulose ; and inorganic powders such as those obtained from aluminum oxide, silica, talc, and zirconium oxide (Cosmetics & Toiletries, Volume 101, July, 1986).

However, with regard to facial cleansers which contain such plant-based, animal-based, or inorganic powders as listed above, the compounded powders are not spherical, but rather have acute angles, and also have a high hardness, and as a consequence thereof, the facial cleanser can damage the skin when applied. Furthermore, the powders of natural origin contain various admixed impurities, which can easily cause spoilage and promote skin irritation.

On the other hand, with regard to facial cleansers which contain organic powders, although the blended cosmetic will have a smooth application sensation when a spherical powder is used, it nevertheless evokes the sensation that a foreign-material is being applied to the skin due to the high hardness of the particles involved. Additionally, because contact with the skin is in the form of point contact, removal of dirt from the skin is unsatisfactory.

The object of the present invention is to solve the above problems by introducing a facial cleanser which will not irritate the skin, which is smooth in its application, and which efficiently removes organic dirt from the skin. The aforesaid object is achieved by a facial cleanser which characteristically contains an organopolysiloxane elastomer powder.

To explain the preceding, the organopolysiloxane elastomer used in the present invention provides the facial cleanser of the present invention with a smooth application sensation and does not irritate the skin. Furthermore, because the particles of the powder are elastic, the removal of organic dirt from the skin is improved because contact with the skin is changed from point contact to a surface contact under the effect of the pressure of application.

Moreover, silicones have recently been compounded into foundation and make-up cosmetics. Silicones have much lower surface tensions than other cosmetic starting materials and thus readily adhere to the skin. Prior facial cleansers which contain non-silicone powders have an unsatisfactory silicone-removal action because these powders lack any affinity for silicones. The organopolysiloxane elastomer powder has affinity for the silicone starting materials compounded in cosmetics and thus can remove silicones adhering on the skin.

No specific restriction exists as to the type of curable organopolysiloxane composition which can serve as starting material for the organopolysiloxane elastomer powder. Examples in this respect are addition reaction-curing organopolysiloxane compositions which cure under platinum metal catalysis by the addition reaction between SiH-containing diorganopolysiloxane and organopolysiloxane having silicon-bonded vinyl groups ; condensation-curing organopolysiloxane compositions which cure in the presence of an organotin compound by a dehydrogenation reaction between hydroxyl-terminated diorganopolysiloxane and SiH-containing diorganopolysiloxane ; condensation-curing organopolysiloxane compositions which cure in the presence of an organotin compound or a titanate ester, by a condensation reaction between an hydroxyl-terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions) ; peroxide-curing organopolysiloxane compositions which thermally cure in the presence of an organoperoxide catalyst ; and organopolysiloxane compositions which are cured by high-energy radiation, such as by gamma-rays, ultraviolet radiation, or electron beams.

Addition reaction-curing organopolysiloxane compositions are preferred for their rapid curing rates and excellent uniformity of curing. A particularly preferred addition reaction-curing organopolysiloxane composition is prepared from :

(A) an organopolysiloxane having at least 2 lower alkenyl groups in each molecule ;

(B) an organopolysiloxane having at least 2 silicon-bonded hydrogen atoms in each molecule ; and

(C) a platinum-type catalyst.

With regard to the above, component (A) is the basic component of the silicone elastomer-generating organopolysiloxane, and curing proceeds by the addition reaction of this component with component (B) under catalysis by component (C). This component (A) must contain at least 2 silicon-bonded lower alkenyl groups

in each molecule : an excellent cured product will not be obtained at fewer than two lower alkenyl groups because a network structure will not be formed. Said lower alkenyl groups are exemplified by vinyl, allyl, and propenyl. While the lower alkenyl groups can be present at any position in the molecule, their presence at the molecular terminals is preferred. The molecular structure of this component may be straight chain, branched straight chain, cyclic, or network, but a straight chain, possibly slightly branched, is preferred. The molecular weight of this component is not specifically restricted, and thus the viscosity may range from low viscosity liquids to very high viscosity gums. In order for the cured product to be obtained in the form of the rubbery elastomer, it is preferred that the viscosity at 25 degrees Centigrade be at least 1 $cm^2/s$ (100 centistokes). These organopolysiloxanes are exemplified by methylvinylsiloxanes, methylvinylsiloxane-dimethylsiloxane copolymers, dimethylvinylsiloxy-terminated dimethylpolysiloxanes, dimethylvinylsiloxy-terminated dimethylsiloxane-methylphenylsiloxane copolymers, dimethylvinylsiloxy-terminated dimethylsiloxane-diphenylsiloxane-methylvinylsiloxane copolymers, trimethylsiloxy-terminated dimethylsiloxane-methylvinylsiloxane copolymers, trimethylsiloxy-terminated dimethylsiloxane-methylphenylsiloxane-methylvinylsiloxane copolymers, dimethylvinylsiloxy-terminated methyl(3,3,3-trifluoropropyl) polysiloxanes, and dimethylvinylsiloxy-terminated dimethylsiloxane-methyl(3,3,3-trifluoropropyl)siloxane copolymers.

Component (B) is an organopolysiloxane having at least 2 silicon-bonded hydrogen atoms in each molecule and is a crosslinker for component (A). Curing proceeds by the addition reaction of the silicon-bonded hydrogen atoms in this component with the lower alkenyl groups in component (A) under catalysis by component (C). This component (B) must contain at least 2 silicon-bonded hydrogen atoms in each molecule in order to function as a crosslinker. Furthermore, the sum of the number of alkenyl groups in each molecule of component (A) and the number of silicon-bonded hydrogen atoms in each molecule of component (B) is to be at least 5. Values below 5 should be avoided because a network structure is then essentially not formed.

No specific restriction exists on the molecular structure of this component, and it may be any of straight chain, branch-containing straight chain and cyclic. The molecular weight of this component is not specifically restricted, but it is preferred that the viscosity at 25 degrees Centigrade be 0.01 to 500 $cm^2/s$ (1 to 50,000 centistokes) in order to obtain good miscibility with component (A). It is preferred that this component be added in a quantity such that the molar ratio between the total quantity of silicon-bonded hydrogen atoms in the instant component and the total quantity of all lower alkenyl groups in component (A) falls within the range of (1.5 : 1) to (20 : 1). It is difficult to obtain good curing properties when this molar ratio falls below 0.5 : 1. When (20 : 1) is exceeded, there is a tendency for the hardness to increase to high levels when the cured product is heated. Furthermore, when an organosiloxane containing substantial alkenyl is supplementarily added for the purpose of, for example, reinforcement, it is preferred that a supplemental addition of the instant SiH-containing component be made in a quantity offsetting these alkenyl groups. This component is concretely exemplified by trimethylsiloxy-terminated methylhydrogenpolysiloxanes, trimethylsiloxy-terminated dimethylsiloxane-methylhydrogensiloxane copolymers, and dimethylsiloxane-methylhydrogensiloxane cyclic copolymers.

Component (C) is a catalyst of the addition reaction of silicon-bonded hydrogen atoms and alkenyl groups, and is concretely exemplified by chloroplatinic acid, possibly dissolved in an alcohol or ketone and this solution optionally aged, chloroplatinic acid-olefin complexes, chloroplatinic acid-alkenylsiloxane complexes, chloroplatinic acid-diketone complexes, platinum black, and carrier-supported platinum.

This component is added preferably at 0.1 to 1,000 weight parts, and more preferably at 1 to 100 weight parts, as platinum-type metal proper per 1,000,000 weight parts of the total quantity of components (A) plus (B). Other organic groups which may be bonded to silicon in the organopolysiloxane forming the basis for the above-described curable organopolysiloxane compositions are, for example, alkyl groups such as methyl, ethyl, propyl, butyl, and octyl ; substituted alkyl groups such as 2-phenylethyl, 2-phenylpropyl, and 3,3,3-trifluoropropyl ; aryl groups such as phenyl, tolyl, and xylyl ; substituted aryl groups such as phenylethyl ; and monovalent hydrocarbon groups substituted by, for example, the epoxy group, the carboxylate ester group and the mercapto group.

Examples of the production of the organopolysiloxane elastomer powder are as follows :
an organopolysiloxane composition as described above (additional-curable, condensation-curable, or peroxide-curable) is mixed with water in the presence of a surfactant (nonionic, anionic, cationic, or amphoteric), and, after mixing to homogeneity in a homomixer, colloid mill, homogenizer and propeller mixer, this is cured by discharge into hot water (temperature at least 50 degrees Centigrade) and is then dried ; the organopolysiloxane composition (addition-curable, condensation-curable, or peroxide-curable) is cured by spraying it directly into a heated current ; the powder is obtained by curing a radiation-curable organopolysiloxane composition by spraying it under high-energy radiation ; the organopolysiloxane composition (addition-curable, condensation-curable, or peroxide-curable) or high energy-curable organopolysiloxane composition is cured, the latter by high-energy radiation, and the product is then pulverized using a known pulverizer such as, for example, a ball mill, atomizer, kneader and roll mill, to thereby form the powder.

From the standpoint of producing a small, spherical powder having a uniform particle size, the preferred method is to mix the organopolysiloxane composition (addition-curable, condensation-curable, or peroxide-curable) with water in the presence of surfactant (nonionic, anionic, cationic, or amphoteric) and then mix this to homogeneity using a homomixer, colloid mill, homogenizer and propeller mixer, followed by curing by discharge into hot water (at least 50 degrees Centigrade) and then drying.

Average particle sizes for this component of at least 3 micrometers up to 3,000 micrometers are preferred in order to impart a cleansing action and smoothness to the facial cleanser of the present invention. Average particle sizes of at least 100 micrometers up to 1,000 micrometers are even more preferred. The removal of organic dirt and silicones will be unsatisfactory at average particle sizes below 3 micrometers, while a foreign-material sensation appears at above 3,000 micrometers.

This component's compounding ratio will vary with the cosmetic's formulation and so is not specifically restricted. However, it is preferably 0.5 wt% to 50 wt% for solid and paste cosmetics and is preferably 0.1 wt% to 30 wt% for cream and emulsion cosmetics.

To prepare the facial cleanser of the present invention, organopolysiloxane elastomer powder as described above is blended and dispersed to homogeneity with the typical starting materials used for facial cleansers. These raw materials for facial cleansers are exemplified by surfactants, emollients, fatty acids, alkali substances, alcohols, esters, humectants, thickeners, and purified water, and these may be used singly or as combinations of two or more.

The surfactants in this case are exemplified by nonionic surfactants such as sorbitan fatty acid esters, glycerol fatty acid esters, decaglycerol fatty acid esters, polyglycerol fatty acid esters, propylene glycolpentaerythritol fatty acid esters, polyoxyethylene sorbitan fatty acid esters, polyoxyethylene sorbitol fatty acid esters, polyoxyethylene glycerol fatty acid esters, polyethylene glycol fatty acid esters, polyoxyethylene alkyl ethers, polyoxyethylene phytosterol-phytostal, polyoxyethylene-polyoxypropylene alkyl ethers, polyoxyethylene castor oil/hydrogenated castor oil, polyoxyethylene lanolin/lanolin alcohol, beeswax derivatives, polyoxyethylene alkylamines/fatty acid amides, polyoxytetramethylene polyglyceryl alkyl ethers, alkyl fatty acid triglycerides, and polyoxyalkylene/dimethylpolysiloxane copolymers ; anionic surfactants such as alkyl sulfates, polyoxyethylene alkyl ether sulfates, N-acylamino acid salts, alkyl phosphates, polyoxyethylene alkyl ether phosphates, and fatty acid salts ; cationic surfactants such as alkylammonium salts and alkylbenzylammonium salts ; and amphoteric surfactants such as betaine acetate, imidazolinium betaine, and lecithin.

The emollients are exemplified by plant oils such as linseed oil, soybean oil, castor oil, and coconut oil ; by animal oils such as egg yolk oil, mink oil, beef tallow, lard, and squalane ; by mineral oils such as ceresin, paraffins, and microcrystalline waxes ; and by silicone oils such as dimethylpolysiloxanes, methylphenylpolysiloxanes, methylhydrogenpolysiloxanes, amino-modified polysiloxanes, cyclic methylpolysiloxanes, cyclic methylphenylpolysiloxanes, cyclic methylhydrogenpolysiloxanes, and epoxy-modified polysiloxanes.

The fatty acids are exemplified by myristic acid, lauric acid, palmitic acid, stearic acid, behenic acid, lanolic acid, isostearic acid, undecylenic acid, hydrogenated animal fatty acids, hydrogenated plant fatty acids, and triple-press fatty acids.

The alkali substances are exemplified by sodium hydroxide, potassium hydroxide, calcium hydroxide, diethanolamine, and triethanolamine.

The alcohols are exemplified by lower alcohols such as ethanol, n-propanol, isopropanol, and butanol ; and by higher alcohols such as lauryl alcohol, cetanol, stearyl alcohol, oleyl alcohol, lanolin alcohol, hydrogenated lanolin alcohol, hexyldecanol, octyldecanol, and behenyl alcohol.

The esters are exemplified by fatty acid esters such as isopropyl myristate, butyl stearate, octyldodecyl neodecanoate and cholesteryl stearate, and by the fatty acid esters of polyhydric alcohols such as propylene glycol didecanoate and glycerol tri-2-ethylhexanoate.

The humectants are exemplified by glycerol, propylene glycol, sorbitol, 1,3-butylene glycol, polyethylene glycol, urea, sodium lactate, sodium pyrrolidonecarboxylate, polypeptides, pyroalluronic acid, and acylamino acids.

The thickening agents are exemplified by natural polymers such as guar gum, carrageenan, alginic acid, gum arabic, tragacanth, pectin, starch, xanthan gum, gelatin, casein, and albumin ; by semi-synthetic polymers such as starch derivatives, guar gum derivatives, locust bean gum derivatives, cellulose derivatives, and alginic acid derivatives ; and by synthetic polymers such as polyvinyl alcohol, polyvinyl pyrrolidone, polyvinyl methacrylate, sodium polyacrylate, and polyethylene glycol. Furthermore, non-silicone powders may also be added to the facial cleanser of the present invention as long as the object of the present invention is not adversely affected. These non-silicone powders are exemplified by plant powders, such as the seeds and powders obtained from the apricot, almond, birch, walnut, peach, corn, sunflower and watermelon, as well as hydrogenated jojoba oil and hydrogenated coconut oil ; powders of animal origin such as powdered crab shell, and powdered eggshell, as well as hydrogenated beef tallow and hydrogenated lard ; organic powders such as those

obtained from polyethylene, nylon, polypropylene, polyvinyl chloride, polystyrene, and cellulose ; as well as inorganic powders such as those obtained from aluminum oxide, silica, talc, and zirconium oxide.

EXAMPLES

The present invention will be explained in the following with reference to illustrative examples. In the examples, the term parts refers to weight parts.

EXAMPLE 1

The following were mixed to homogeneity : 100 parts dimethylvinylsiloxy-terminated dimethylpolysiloxane having the following formula :

$$CH_2 = CH(CH_3)_2SiO\ (\{CH_3\}_2SiO)_{60}\ Si(CH_3)_2CH = CH_2,$$

3.4 parts trimethylsilxoy-terminated methylhydrogenpolysiloxane having the following formula :

$$(CH_3)_3SiO\ (\{CH_3\}HSiO)_{25}\ Si(CH_3)_3,$$

0.3 parts polyoxyethylene (9EO) lauryl ether, and isopropanolic chloroplatinic acid in a quantity sufficient to give 100 ppm platinum metal proper based on the total quantity of organopolysiloxane. 100 Parts purified water was added and this was then passed through a colloid mill (colloid gap = 1 mm) and discharged into hot water (90 degrees Centigrade) to produce an organopolysiloxane elastomer powder. Using scanning electron microscopy, this organopolysiloxane elastomer powder was found to be spherical and to have an average particle diameter of 250 micrometers. Furthermore, it was elastic.

A cleansing cream (foam) with the composition given in Table 1 was prepared using this organopolysiloxane elastomer powder. This cleansing cream was prepared as follows. Glycerol, potassium hydroxide, and water were mixed in advance at 70 degrees Centigrade, and this mixture was dripped into a mixture (heated at 70 degrees Centigrade) of stearic acid, palmitic acid, myristic acid, lauric acid, oleyl alcohol, lanolin-EO adduct and the organopolysiloxane elastomer powder, followed by cooling to room temperature (25 degrees Centigrade) upon the completion of addition.

Comparative Examples 1 and 2 consisted, respectively, of this same formulation, either containing the same amount of polyethylene powder (average particle diameter = 150 micrometers) in place of the organopolysiloxane elastomer powder or lacking any powder.

Collagen membranes were prepared in advance by coating a 7 cm diameter circular area with 0.1 g foundation having the composition given in Table 2. In each case, 0.1 g cleansing cream was applied and rubbed uniformly over the foundation region for 30 seconds using a finger, followed by wiping with a water-soaked towel for 10 seconds and then wiping with a dry towel.

In order to determined the cleansing effect of each cleansing cream, the brightness and angle of contact for water were determined on the cleaned collagen membrane respectively using a CR100 color-difference colorimeter from Minolta Camera Kabushiki Kaisha and a contact-angle measurement device from Erma Optical Works Limited.

Sensory testing of the application of each cleansing foam was conducted using a ten-member panel. The various results are reported in Table 3.

TABLE 1

| component | Example 1 (parts) | Comparative Example 1 (parts) | Comparative Example 2 (parts) |
|---|---|---|---|
| stearic acid | 10.0 | 10.0 | 10.0 |
| palmitic acid | 10.0 | 10.0 | 10.0 |
| myristic acid | 10.0 | 10.0 | 10.0 |
| lauric acid | 6.0 | 6.0 | 6.0 |
| oleyl alcohol | 1.5 | 1.5 | 1.5 |
| lanolin-EO adduct | 1.0 | 1.0 | 1.0 |
| organopolysiloxane elastomer powder | 5.0 | 0.0 | 0.0 |
| polyethylene powder | 0.0 | 5.0 | 0.0 |
| glycerol | 18.0 | 18.0 | 18.0 |
| potassium hydroxide | 3.5 | 3.5 | 3.5 |

TABLE 2

| FORMULATION OF FOUNDATION USED IN EVALUATION OF CLEANSING EFFECT | |
|---|---|
| components | quantity in parts |
| silicone-treated titanium oxide (AS61D from Toshiki Pigment Kabushiki Kaisha) | 40 |
| dimethylpolysiloxane/2cm²/s (200 cst) | 20 |
| decamethylcyclopentasiloxane | 30 |
| liquid paraffin (mp 70 degrees Centigrade) | 20 |
| silicon dioxide (R972 from Nippon Aerosil Kabushiki Kaisha) | 10 |

TABLE 3

| RESULTS OF THE EVALUATIONS | | | |
|---|---|---|---|
| | Example 1 | Comparative Example 1 | Comparative Example 2 |
| brightness | 50.1 | 55.3 | 58.9 |
| contact angle | 87° | 95° | 103° |
| sensory testing (number of panelists) | | | |
| too greasy | - | - | 5 |
| smooth | 4 | - | 5 |
| foreign-material sensation | 6 | 3 | - |
| irritating | - | 7 | - |

EXAMPLE 2

A liquid mixture was prepared from 100 parts hydroxyl-terminated dimethylpolysiloxane having the following formula :

$$HO(\overset{CH_3}{\underset{CH_3}{Si}}O)_{120}H$$

100 parts trimethylsiloxy-terminated methylhydrogenpolysiloxane having the following formula :

$$(CH_3)_3SiO(\{CH_3\}HSiO)_{25}Si(CH_3)_3,$$

1 part stannous octoate, 1 part polyoxyethylene (8EO) oleyl ether, and 1,000 parts ion-exchanged water. This was then emulsified in a colloid mill, heated at 50 degrees Centigrade for 1 hour, and then spray-dried to produce an organopolysiloxane elastomer powder. Using scanning electron microscopy, this organopolysiloxane elastomer powder was found to be spherical and to have an average particle diameter of 150 micrometers. Furthermore, it was elastic.

A cleansing cream with the composition given in Table 4 was prepared as follows using this organopolysiloxane elastomer powder. Hot water (70 degrees Centigrade) was dripped into a melt-mixture (70 degrees Centigrade) prepared from the solid paraffin, beeswax, vaseline, liquid paraffin, sorbitan sesquiisostearate, POE (20EO) sorbitan monooleate, and organopolysiloxane elastomer powder.

Comparative Examples 3 and 4 consisted, respectively, of this same formulation in which the organopolysiloxane elastomer powder was replaced by an equal quantity of polyethylene powder (average particle diameter = 350 micrometers), and the above formulation lacking any powder.

The cleansing effect determination and sensory testing were conducted on each cleansing cream as described in Example 1, and the results of these evaluations are reported in Table 5.

TABLE 4

| component | Example 2 (parts) | Comparative Example 3 (parts) | Comparative Example 4 (parts) |
|---|---|---|---|
| solid paraffin | 10 | 10 | 10 |
| beeswax | 3 | 3 | 3 |
| petrolatum (vaseline®) | 15 | 15 | 15 |
| liquid paraffin | 41 | 41 | 41 |
| sorbitan sesquiisostearate | 4.2 | 4.2 | 4.2 |
| POE (20EO) sorbitan monooleate | 0.8 | 0.8 | 0.8 |
| organopolysiloxane elastomer powder | 5 | - | - |
| polyethylene powder | - | 5 | - |
| water | 20.5 | 20.5 | 20.5 |

TABLE 5

| RESULTS OF THE EVALUATIONS | | | |
|---|---|---|---|
| | Example 2 | Comparative Example 3 | Comparative Example 4 |
| brightness | 48.1 | 50.0 | 55.2 |
| contact angle | 85° | 87° | 86° |
| sensory testing (number of panelists) | | | |
| too greasy | - | - | 8 |
| smooth | 8 | - | 2 |
| foreign-material sensation | 2 | 1 | - |
| irritating | - | 9 | - |

EXAMPLE 3

A cleansing lotion with the composition given in Table 6 was prepared using the organopolysiloxane elastomer powder prepared in Example 1.

The cleansing lotion was prepared by dripping ion-exchanged water with stirring into a liquid mixture of silicone surfactant (DC3225C from Toray Silicone Company Limited), decamethylcyclopentasiloxane, the organopolysiloxane elastomer powder and POE (2OEO) sorbitan monolaurate. After confirmation of a homogeneous mixture, mixing was continued in a homomixer to obtain the cleansing lotion.

Comparative Examples 5 and 6 consisted, respectively, of the same formulation as above in which the organopolysiloxane elastomer powder was replaced by the same quantity of polyethylene powder (average particle size = 20 micrometers), and the same formulation as above lacking any powder.

The cleansing effect determination and sensory testing were conducted as described in Example 1.

The results of these evaluations are reported in Table 7.

TABLE 6

| component | Example 3 | Comparative Example 5 | Comparative Example 6 |
|---|---|---|---|
| | (parts) | (parts) | (parts) |
| silicone surfactant (DC3225C) | 9.5 | 9.5 | 9.5 |
| decamethylcyclo-pentasiloxane | 10 | 10 | 10 |
| organopolysiloxane elastomer powder | 3 | - | - |
| polyethylene powder | - | 3 | - |
| POE (20EO) sorbitan monolaurate | 0.5 | 0.5 | 0.5 |
| ion-exchanged water | 74.5 | 74.5 | 74.5 |

TABLE 7

| RESULTS OF THE EVALUATIONS | | | |
|---|---|---|---|
| | Example 3 | Comparative Example 5 | Comparative Example 6 |
| brightness | 44.1 | 48.8 | 53.3 |
| contact angle | 58° | 63° | 68° |
| sensory testing (number of panelists) | | | |
| too greasy | - | 6 | - |
| smooth | 8 | 4 | 10 |
| foreign material sensation | 2 | - | - |

The cleanser of the present invention, because it contains an organopolysiloxane elastomer powder, characteristically will not irritate the skin, is smooth in its application, and effectively removes organic dirt and silicones from the skin.

## Claims

1. A facial cleanser having the characteristic that it contains organopolysiloxane elastomer powder.

2. A facial cleanser described in Claim 1 wherein the average particle size of the organopolysiloxane elastomer powder is 3 micrometers to 3,000 micrometers.

3. A facial cleanser described in Claim 1 wherein the organopolysiloxane elastomer powder is spherical.

4. A facial cleanser described in Claim 1 wherein the content of organopolysiloxane elastomer powder is 0.5 to 50 wt%.

## Patentansprüche

1. Gesichtsreinigungsmittel, **dadurch gekennzeichnet**, daß es ein pulverförmiges Organopolysiloxanelastomer enthält.

2. Gesichtsreinigungsmittel nach Anspruch 1, **dadurch gekennzeichnet**, daß die mittlere Teilchengröße des pulverförmigen Organopolysiloxanelastomeren 3-3000 μm beträgt.

3. Gesichtsreinigungsmittel nach Anspruch 1, **dadurch gekennzeichnet**, daß das pulverförmige Organopolysiloxanelastomer kugelförmig ist.

4. Gesichtsreinigungsmittel nach Anspruch 1, **dadurch gekennzeichnet**, daß der Gehalt pulverförmigen Organopolysiloxanelastomers 0,5-50 Gew.-% beträgt.

## Revendications

1. Un produit de nettoyage facial caractérisé en ce qu'il contient une poudre d'élastomère d'organopolysiloxane.

2. Un produit de nettoyage facial, selon la revendication 1, dans lequel la grosseur de particule moyenne de la poudre d'élastomère d'organopolysiloxane est de 3 micromètres à 3000 micromètres.

3. Un produit de nettoyage facial selon la revendication 1, dans lequel la poudre d'élastomère d'organopolysiloxane est sphérique.

4. Un produit de nettoyage facial selon la revendication 1, dans lequel la teneur en poudre d'élastomère d'organopolysiloxane est de 0, 5 à 50% en poids.